# EUROPÄISCHE PATENTANMELDUNG

(11) **EP 2 939 698 A1**
(43) Veröffentlichungstag der Anmeldung: **04.11.2015**
(21) Anmeldenummer: 14166442.5
(22) Anmeldetag: 29.04.2014
(51) Int. Cl.: A61M 1/10, A61M 1/12

(54) **Implantierbare Anordnung und Verfahren zum Herstellen einer implantierbaren Anordnung**

(71) Anmelder: Berlin Heart GmbH, 12247 Berlin (DE)
(72) Erfinder: Kaebe, Benjamin, 14199 Berlin (DE); Czogalla, Frank, 14513 Teltow (DE)
(74) Vertreter: Pfenning, Meinig & Partner GbR

(57) **Zusammenfassung**

Die Erfindung betrifft ein Verfahren zum Herstellen einer implantierbaren Anordnung (3) zum Leiten einer Flüssigkeit, insbesondere von Blut, bei dem ein biokompatibles oder biokompatibel umhülltes erstes Hohlelement (10) und ein biokompatibles oder biokompatibel umhülltes zweites Hohlelement (11) sowie mindestens ein Verbindungselement (13, 14) zum Verbinden des ersten Hohlelements (10) mit dem zweiten Hohlelement (11) bereitgestellt werden, wobei das mindestens eine Verbindungselement (13, 14) zumindest bereichsweise aus einem Formgedächtnismaterial (22) besteht, wobei das erste Hohlelement (10), das zweite Hohlelement (11) und das mindestens eine Verbindungselement (13, 14) so zusammengesetzt werden, dass ein erster Hohlraum des ersten Hohlelements (10) und ein zweiter Hohlraum des zweiten Hohlelement (11) einen Fließkanal (18) für die Flüssigkeit ausbilden und das mindestens eine Verbindungselement (13, 14) mit dem ersten Hohlelement (10) und/oder mit dem zweiten Hohlelement (11) axial überlappt, wobei anschließend ein Formgedächtnis des Formgedächtnismaterials (22) des mindestens einen Verbindungselementes (13, 14) aktiviert wird. Die Erfindung betrifft außerdem eine implantierbare Anordnung (3) sowie ein Blutpumpensystem.

## Beschreibung

Die Erfindung betrifft ein Verfahren zum Herstellen einer implantierbaren Anordnung zum Leiten einer Flüssigkeit, insbesondere von Blut, sowie eine implantierbare Anordnung zum Leiten einer Flüssigkeit, insbesondere Blut, und ein Blutpumpensystem.

Bei der Herstellung einer implantierbaren Anordnung zum Leiten einer Flüssigkeit, wie etwa einer Körperflüssigkeit, insbesondere von Blut, werden in der Regel zwei oder mehr Hohlelemente (d.h. Hohlkörper) derart zusammengesetzt, dass Hohlräume dieser Hohlelemente einen durchgängigen und möglichst flüssigkeitsdichten Fließkanal für die Flüssigkeit ausbilden. Eines der Hohlelemente kann beispielsweise eine Gefäßprothese sein, im Nachfolgenden auch als "Graft" bezeichnet, welche bei einer Implantation der Anordnung in einen lebenden Körper mit einem Gefäß des Körpers, beispielsweise mit einem Blutgefäß, vernäht oder auf andere Weise verbunden wird und beispielsweise aus einem (gewebten) Polyester oder einem anderen biokompatiblen Material bestehen kann. Ein anderes der Hohlelemente kann beispielsweise aus Silikon gefertigt sein. Es kann als ein Schlauch oder eine biegeweiche oder biegestarre Kanüle oder als ein Rohrstück, Rohrstutzen, Hülse oder Olive ausgestaltet sein. Dieses Hohlelement kann beispielsweise an einem Ende eine Kupplungsvorrichtung aufweisen, über die es mit einer ebenfalls implantierbaren oder externen Blutpumpe verbindbar ist, oder es kann fest mit der Blutpumpe verbunden bzw. ein Teil der Blutpumpe sein. Dieses Hohlelement kann also insbesondere als eine Auslasskanüle oder eine Einlasskanüle einer externen oder implantierbaren Blutpumpe oder als ein Einlass oder als ein Auslass einer solchen Blutpumpe ausgestaltet sein.

Bei einer Implantation der Anordnung ist bzw. wird typischerweise zunächst eines der Hohlelemente der Anordnung mit einer typischerweise bereits implantierten Blutpumpe verbunden, beispielsweise wenn es sich bei diesem Hohlelement um einen Ein- oder Auslass der Blutpumpe handelt, bevor dieses Hohlelement nachfolgend mit einem anderen Hohlelement der Anordnung, beispielsweise mit einer Gefäßprothese, verbunden wird. Oftmals ist es notwendig, dieses mit der Blutpumpe verbundene Hohlelement zunächst zu kürzen, um es an die gegebenen anatomischen Strukturen und vorliegende anatomische Größenverhältnisse anzupassen, und es erst anschließend mit dem anderen Hohlelement zu verbinden. Die Herstellung dieser Verbindung ist oftmals schwierig und/oder zeitaufwendig, da das gekürzte Hohlelement an seinem gekürzten Ende in der Regel keine eigenen Verbindungs- oder Kupplungsstrukturen oder -elemente aufweist, die eine einfache und schnelle Verbindungsherstellung der Hohlelemente ermöglichen würden.

Das Herstellen einer möglichst haltbaren und flüssigkeitsdichten Verbindung zwischen den Hohlelementen implantierbarer Anordnungen, wie den oben beschriebenen, stellt oftmals eine Herausforderung dar, sei es aufgrund einer vorgegebenen Abfolge von Verfahrensschritten, beispielsweise bei einer Implantation der Anordnung, sei es aufgrund besonderer mechanischer Eigenschaften bzw. Verschiedenartigkeiten der zu verbindenden Hohlelemente oder sei es aufgrund eines hohen Zeitdrucks oder eines nur sehr kleinen für die Anordnung zur Verfügung stehenden Raums.

Es stellt sich somit die Aufgabe, ein Verfahren zum Herstellen einer möglichst haltbaren und flüssigkeitsdichten implantierbaren Anordnung, beispielsweise der oben beschriebenen Art, vorzuschlagen, wobei sich das Verfahren insbesondere möglichst einfach und schnell durchführen lassen soll. Es soll außerdem eine implantierbare Anordnung der oben beschriebenen Art sowie ein Blutpumpensystem mit einer solchen Anordnung vorgeschlagen werden, welches sich möglichst einfach herstellen bzw. verbinden lässt und möglichst dicht und haltbar ist.

Zur Lösung dieser Aufgabe wird das Verfahren gemäß Anspruch 1, die Anordnung gemäß Anspruch 14 und das Blutpumpensystem gemäß Anspruch 16 vorgeschlagen. Weiterbildungen und spezielle Ausführungsformen des vorgeschlagenen Verfahrens, der vorgeschlagenen Anordnung und des vorgeschlagenen Blutpumpensystems sind Gegenstände der übrigen Ansprüche sowie der nachfolgenden Beschreibung.

Bei dem hier vorgeschlagenen Verfahren zum Herstellen einer implantierbaren Anordnung der oben beschriebenen Art zum Leiten einer Flüssigkeit, insbesondere von Blut oder anderen Körperflüssigkeiten, wird somit zunächst ein biokompatibles erstes Hohlelement und ein biokompatibles zweites Hohlelement sowie mindestens ein Verbindungselement zum Verbinden des ersten Hohlelements mit dem zweiten Hohlelement bereitgestellt. Beispielsweise kann zur Gewährleistung der Biokompatibilität das erste und/oder das zweite Hohlelement (und/oder, sofern vorhanden, das dritte Hohlelement, siehe unten) aus einem biokompatiblen Material, wie beispielsweise Titan, Silikon oder Polyester(-gewebe), gefertigt sein oder (ggf. zusätzlich) biokompatibel beschichtet oder umhüllt sein, also beispielsweise eine Beschichtung oder Umhüllung aus einem biokompatiblen Material, wie etwa Silikon, aufweisen, die den jeweiligen Hohlkörper teilweise oder ringsum vollständig (also hermetisch) umschließt. Das mindestens eine Verbindungselement, welches also ein, zwei oder mehr Verbindungselemente umfassen kann, besteht vollständig oder zumindest bereichsweise aus einem Formgedächtnismaterial, wie beispielsweise Nitinol. Das erste Hohlelement, das zweite Hohlelement und das mindestens eine Verbindungselement werden so zusammengesetzt, dass ein erster Hohlraum des ersten Hohlelements und ein zweiter Hohlraum des zweiten Hohlelements einen Fließkanal für die Flüssigkeit ausbilden. Die Hohlräume des ersten und des zweiten Hohlelements bilden somit axiale Abschnitte dieses Fließkanals, welche beispielsweise direkt aneinander angrenzen bzw. ineinander übergehen oder beispielsweise über einen Hohlraum eines dritten Hohlelementes (häufig auch als Konnektor bezeichnet) miteinander verbunden sind. Das mindestens eine Verbindungselement wird so angeordnet, dass es mit dem ersten Hohlelement und/oder mit dem zweiten Hohlelement axial überlappt.

Hierbei bezieht sich der Ausdruck "axial" jeweils auf die Längsachse des Fließkanals der Anordnung, also auf die Längsausdehnung des Fließkanals bzw. auf eine bestimmungsgemäße Hauptfließrichtung der Flüssigkeit durch den Fließkanal entlang der Längsausdehnung des Fließkanals. Zwei Elemente überlappen sich somit axial, wenn sie sich in einer Blickrichtung senkrecht zur Längsachse des Fließkanals gesehen gegenseitig überlappen, wenn also in dieser Blickrichtung gesehen eines dieser Elemente das andere überdeckt. Entsprechend beziehen sich auch die Begriffe "radial" und "azimutal" auf die Längsachse des Fließkanals. Sofern im Folgenden das Überlappen von Elementen beschrieben wird, ist damit in der Regel dieses axiale Überlappen der Elemente gemeint.

Nach dem Zusammensetzen des ersten und des zweiten Hohlelements wird ein Formgedächtnis des Formgedächtnismaterials des mindestens einen Verbindungselementes aktiviert. Aufgrund dieser Aktivierung baut sich in dem Formgedächtnismaterial eine mechanische Spannung auf, welche ihrerseits ein Verformen des Formgedächtnismaterials und somit auch ein Verformen des mindestens einen Verbindungselements bewirkt, beispielsweise ein (beispielsweise radiales) Verengen oder ein (beispielsweise radiales) Aufweiten des jeweiligen Verbindungselements, insbesondere wenn dieses als eine Hülse oder als ein Ring ausgestaltet ist, wie weiter unten detaillierter beschrieben wird.

Durch diese Verformung werden typischerweise (beispielsweise ringförmige) Lücken, Spalte bzw. Zwischenräume zwischen dem mindestens einen Verbindungselement und dem ersten Hohlelement und/oder dem zweiten Hohlelement geschlossen. In diesem Fall bewegt sich das mindestens eine Verbindungselement durch seine Verformung in Richtung des ersten und/oder des zweiten Hohlelements, d.h. beispielsweise radial nach innen oder radial nach außen. Typischerweise entsteht und/oder verstärkt sich durch diese Verformung ein mechanischer Kontakt bzw. eine mechanische Kopplung zwischen dem Verbindungselement und dem ersten Hohlelement und/oder dem zweiten Hohlelement. Dieser Kontakt bzw. Kopplung kann direkt sein (durch eine gegenseitige Berührung des Verbindungselementes und des ersten Hohlelementes und/oder des zweiten Hohlelementes) oder indirekt sein (über ein zwischen dem Verbindungselement und dem ersten Hohlelement und/oder dem zweiten Hohlelement angeordnetes Zwischenelement, wie beispielsweise eine Silikonhülse oder Ähnliches, siehe unten). Über diesen Kontakt bzw, diese Kopplung überträgt das mindestens eine Verbindungselement die durch die Aktivierung hervorgerufene mechanische Spannung des Formgedächtnismaterials auf das erste Hohlelement und/oder auf das zweite Hohlelement und ruft in dem jeweiligen Hohlelement eine entgegengesetzte mechanische Spannung hervor. Die Verformung setzt sich typischerweise soweit fort, bis die durch das erste bzw. das zweite Hohlelement oder ggf. dritte Hohlelement erzeugte mechanische (Gegen-)Spannung die von dem Formgedächtnismaterial erzeugte Spannung kompensiert und durch das erste bzw. das zweite oder ggf. dritte Hohlelement eine weitere Verformung des Verbindungselements verhindert wird. In diesem Zustand presst das mindestens eine Verbindungselement das erste Hohlelement und/oder das zweite Hohlelement und/oder das dritte Hohlelement mit den nun vorliegenden mechanischen Spannungen gegeneinander, so dass die Hohlelemente auf diese Weise miteinander verbunden sind, typischerweise durch einen Kraftschluss und/oder durch einen (zusätzlichen) Formschluss.

Typischerweise überträgt das mindestens eine Verbindungselement vor der Aktivierung und der Verformung des Formgedächtnismaterials keine oder nur eine sehr geringe Spannung auf die Hohlelemente, so dass es beim Zusammensetzen der Anordnung relativ zu dem ersten und/oder dem zweiten Hohlelement (insbesondere in axialer Richtung) leicht verschoben werden kann, insbesondere auf manuelle Weise. Hierzu können beispielsweise zunächst das erste Hohlelement und das zweite Hohlelement (und ggf. das dritte Hohlelement) relativ zueinander bis zu einer Endausrichtung ausgerichtet werden. Anschließend kann in diesem Beispiel, während das erste Hohlelement und das zweite Hohlelement (und ggf. das dritte Hohlelement) in der Endausrichtung verbleiben bzw. gehalten werden, das mindestens eine Verbindungselement relativ zum ersten Hohlelement und relativ zum zweiten Hohlelement (und ggf. relativ zum dritten Hohlelement) bis zu einer axialen Endposition des mindestens einen Verbindungselements axial verschoben werden, beispielsweise durch manuelles Verschieben des mindestens einen Verbindungselements relativ zu den Hohlelementen.

Durch das Übertragen der mechanischen Spannung des Formgedächtnismaterials auf das erste und/oder das zweite Hohlelement bzw. durch das Pressen des mindestens einen Verbindungselements auf das erste und/oder zweite Hohlelement werden die Hohlelemente miteinander und, sofern vorhanden, auch mit dem dritten Hohlelement verbunden. Falls beispielsweise zwei Verbindungselemente verwendet werden, überlappt eines der beiden Verbindungselemente typischerweise das erste Hohlelement axial und überlappt das andere Verbindungselement typischerweise das zweite Hohlelement axial. Typischerweise umfasst die Anordnung in diesem Fall außerdem das genannte dritte Hohlelement und überlappen beide Verbindungselemente jeweils dieses dritte Hohlelement axial.

Die durch das Formgedächtnismaterial erzeugte und auf die Hohlelemente übertragene Spannung und das Pressen des mindestens einen Verbindungselementes auf das erste und/oder zweite Hohlelement kann prinzipiell für eine unbegrenzte Dauer bestehen bzw. erfolgen, beispielsweise für eine Dauer von Tagen, Wochen, Monaten oder Jahren, sofern keine entgegenwirkenden Maßnahmen ergriffen werden, so dass die hierdurch bewirkte Verbindung zwischen den Hohlelementen für die entsprechende Dauer besteht. Typischerweise wird nach dem Aktivieren des Formgedächtnisses des mindestens einen Verbindungselementes die Anordnung durch das mindestens eine Verbindungselement stabilisiert und die Hohlelemente und das mindestens eine Verbindungselement in ihrer relativen Anordnung relativ zueinander fixiert, so dass die während der Aktivierung bestehende relative Ausrichtung des mindestens einen Verbindungselements und der Hohlelemente dauerhaft erhalten bleibt. Somit zeichnet sich eine unter Anwendung des hier vorgeschlagenen Herstellungsverfahrens hergestellte implantierbare Anordnung insbesondere auch durch die während des Aktivierens des Formgedächtnismaterials bestehende relative Ausrichtung des mindestens einen Verbindungselements und der Hohlelemente aus.

Beispielsweise können das erste Hohlelement und das zweite Hohlelement so zusammengesetzt werden, dass sie sich in einem Überlappungsbereich axial überlappen, wobei das mindestens eine Verbindungselement in diesem Überlappungsbereich angeordnet wird. Nach dem Aktivieren des Formgedächtnisses werden auf die oben beschriebene Weise das erste Hohlelement und das zweite Hohlelement mittels des mindestens einen Verbindungselements aufeinandergepresst. In diesem Fall reicht ein einziges Verbindungselement oftmals aus, um die Hohlelemente miteinander zu verbinden. Dieses überlappt typischerweise sowohl das erste als auch das zweite Hohlelement (und ggf. auch ein drittes Hohlelement) axial.

Wie oben bereits erwähnt worden ist, kann zusätzlich ein drittes Hohlelement bereitgestellt werden und das erste Hohlelement, das zweite Hohlelement und dritte Hohlelement so zusammengesetzt werden, dass ein dritter Hohlraum des dritten Hohlelements mit dem ersten Hohlraum des ersten Hohlelements und dem zweiten Hohlraum des zweiten Hohlelements den genannten Fließkanal ausbildet. Außerdem werden diese Hohlelemente hierbei so zusammengesetzt, dass das dritte Hohlelement sowohl mit dem ersten Hohlelement als auch mit dem zweiten Hohlelement in mindestens einem Überlappungsbereich (typischerweise in einem Überlappungsbereich oder in zwei Überlappungsbereichen) axial überlappt. Das mindestens eine Verbindungselement wird in diesem mindestens einen Überlappungsbereich angeordnet. Falls beispielsweise zwei Verbindungselemente verwendet werden, überlappt eines dieser Verbindungselemente typischerweise das erste Hohlelement und das dritte Hohlelement in deren Überlappungsbereich axial und überlappt das andere Verbindungselement typischerweise das zweite Hohlelement und das dritte Hohlelement in deren Überlappungsbereich axial. Die genannten Überlappungsbereiche können voneinander axial beabstandet sein, d.h. voneinander axial getrennt angeordnet sein. Nach dem Aktivieren des Formgedächtnismaterials des mindestens einen Verbindungselements werden durch das mindestens eine Verbindungselement das erste Hohlelement und das dritte Hohlelement auf die oben beschriebene Weise aufeinandergepresst und werden entsprechend auch das zweite Hohlelement und das dritte Hohlelement aufeinandergepresst.

Das erste Hohlelement und das zweite Hohlelement erstrecken sich in axialer Richtung hauptsächlich (d.h. größtenteils) außerhalb der genannten Überlappungsbereiche. Typischerweise sind die axialen Gesamtlängen des ersten Hohlelements und des zweiten Hohlelements wesentlich größer als die axiale Ausdehnung der genannten Überlappungsbereiche, typischerweise um mehr als das Fünffache oder sogar um mehr als das Zehnfache. Dies bedeutet, dass weniger als 20% oder sogar weniger als 10% der jeweiligen axialen Gesamtausdehnung des ersten bzw. zweiten Hohlelements innerhalb des jeweiligen Überlappungsbereichs angeordnet ist. Dahingegen erstreckt sich das dritte Hohlelement, sofern vorhanden, in axialer Richtung hauptsächlich innerhalb der genannten Überlappungsbereiche. Typischerweise wird das dritte Hohlelement vollständig oder zumindest zu einem großen Teil von dem ersten und dem zweiten Hohlelement axial überlappt, so dass die axiale Gesamtlänge des dritten Hohlelements typischerweise zu mindestens 50% oder sogar zu mindestens 80 % oder sogar zu 100% axial mit dem ersten und/oder zweiten Hohlelement axial überlappt. Das mindestens eine Verbindungselement, oder zumindest dessen Formgedächtnismaterial, ist typischerweise vollständig innerhalb des jeweiligen Überlappungsbereichs angeordnet, kann aber auch axial über das erste bzw. das zweite Hohlelement hinausragen.

Vorzugsweise bildet das Formgedächtnismaterial des mindestens einen Verbindungselements mindestens einen Ring, der vorzugsweise ringsum (d.h. in azimutaler Richtung) geschlossen ist, oder mindestens eine Hülse, die vorzugsweise ringsum (d.h. in azimutaler Richtung) geschlossen ist, wobei der mindestens eine Ring bzw. die mindestens eine Hülse den Fließkanal (azimutal) umläuft. Typischerweise ist das Formgedächtnismaterial, wie vorzugsweise auch das mindestens eine Verbindungselement, jeweils hohlzylindrisch ausgestaltet. Die genannten Hohlelemente sind ebenfalls vorzugsweise hohlzylindrisch bzw. schlauchartig ausgestaltet.

Durch die Aktivierung des Formgedächtnisses des Formgedächtnismaterials wird typischerweise eine Phasenumwandlung des Formgedächtnismaterials ausgelöst, welche daraufhin vollständig oder unvollständig, d.h. nur teilweise bzw. bereichsweise, ablaufen kann, so dass die durch das Formgedächtnismaterial hervorgerufene und auf die Hohlelemente übertragene mechanische Spannung (zumindest teilweise) dauerhaft erhalten bleibt. Beispielsweise ist es möglich, dass die Phasenumwandlung deswegen nur teilweise erfolgt, weil die durch das erste und/oder zweite Hohlelement ausgeübte mechanische (Gegen-)Spannung (wie oben beschrieben wurde) eine vollständige Phasenumwandlung verhindert. Eine solche unvollständige Phasenumwandlung ist im Zusammenhang mit Formgedächtnismaterialien beispielsweise unter dem Begriff "unterdrücktes Formgedächtnis" bekannt. Typischerweise handelt es sich bei der Phasenumwandlung um eine diffusionslose Phasenumwandlung, beispielsweise um eine sogenannte "martensitische Transformation". Bei der Phasenumwandlung nach dem Aktivieren des Formgedächtnisses des Formgedächtnismaterials kann beispielsweise eine atomare Struktur des Formgedächtnismaterials, wie beispielsweise eine atomare Gitterstruktur, vollständig oder zumindest bereichsweise von einer martensitischen (Gitter-)Struktur in eine austenitische (Gitter-)Struktur übergehen. Beispielsweise kann sich das Formgedächtnismaterial (vollständig oder nicht-vollständig) von einer Niedertemperaturphase in eine Hochtemperaturphase umwandeln und/oder von einer martensitischen Phase (bzw. von einem Martensit) in eine austenitischen Phase (bzw. in einen Austenit) oder jeweils umgekehrt. In manchen Fällen wird ein möglichst vollständiger Übergang angestrebt.

Insbesondere ist es mit dem vorgeschlagenen Verfahren möglich, den im Zusammenhang mit Formgedächtnismaterialien bekannten "Einweg(-Memory-) effekt" auszunutzen, wodurch vorteilhafterweise besonders große mechanische Spannungen hervorgerufen werden können. Hierzu wird, typischerweise bereits vor dem Zusammensetzen des mindestens einen Verbindungselements mit dem ersten Hohlelement und mit dem zweiten Hohlelement, das Formgedächtnismaterial in der martensitischen Phase und ausgehend von einer äußeren Ausgangsform des Formgedächtnismaterials durch Einbringen äußerer Kräfte vorzugsweise bis zu einer teilweisen oder möglichst vollständigen "Entzwillingung" des (zunächst "verzwillingten") Formgedächtnismaterials (typischerweise elastisch) verformt und hierbei in eine äußere Zwischenform des Formgedächtnismaterials gebracht. Typischerweise erfolgt dies unterhalb der sogenannten Martensit-Start-Temperatur (Mₛ) oder unterhalb der sogenannten Martensit-End-Temperatur (Mₑ) des Formgedächtnismaterials. Nach der Aktivierung des Formgedächtnisses in der verformten ("entzwillingten") martensitischen Phase, typischerweise durch eine ausreichende Erwärmung des Formgedächtnismaterials, erfolgt die weiter oben beschriebene eigenständige Verformung des Formgedächtnismaterials durch eine (teilweise oder vollständige) Transformation zurück in die ("verzwillingte") austenitische Phase zurück zur Ausgangsform. Die Ausgangsform wird typischerweise nur dann vollständig erreicht, wenn diese Phasenumwandlung vollständig abläuft. Andernfalls nähert sich das Formgedächtnismaterial der Ausgangsform so weit an, bis ein weitergehender Ablauf des Phasenübergangs unterdrückt wird, typischerweise durch die oben beschriebene (Gegen-)Spannung des ersten und/oder zweiten Hohlelementes. Das Formgedächtnis des Formgedächtnismaterials kann, je nach gewähltem Formgedächtnismaterial, durch Erwärmen des Formgedächtnismaterials auf (oder über) eine Aktivierungstemperatur des Formgedächtnismaterials aktiviert werden, beispielsweise auf (oder über) die sogenannte Austenit-Start-Temperatur (Aₛ) oder auf (oder über) die sogenannten Austenit-End-Temperatur (Aₑ) des Formgedächtnismaterials.

Anstelle des "Einweg(-Memory-)effektes" kann auch der für Formgedächtnismaterialien ebenfalls allgemein bekannte "Zweiweg(-Memory-)effekt" ausgenutzt werden. Während beim Einwegeffekt das Formgedächtnis typischerweise dadurch ausgebildet wird, dass das Formgedächtnismaterial ein einziges Mal auf die oben beschriebene Weise in die martensitische Phase überführt ("entzwillingt") wird, wird das Formgedächtnis im Fall des Zweiwegeffektes in mehreren thermomechanischen Behandlungszyklen trainiert, was typischerweise noch vor dem Zusammensetzen des mindestens einen Verbindungselements mit dem ersten Hohlelement und mit dem zweiten Hohlelement erfolgt. Hierfür wird das Formgedächtnismaterial auf im Prinzip bekannte Art und Weise mehrmals unter bestimmten äußeren Bedingungen zwischen zwei Zielformen hin und her verformt. Beispielsweise kann das Formgedächtnis des Formgedächtnismaterial derart trainiert sein, dass sich das Formgedächtnismaterial nach einer entsprechenden Aktivierung seines Formgedächtnisses eigenständig zwischen zwei Phasen bzw. zwischen zwei (die innere atomare Struktur des Formgedächtnismaterials betreffenden) Zuständen umwandelt und sich hierbei jeweils, wie oben beschrieben, verformt, so dass jedem dieser beiden Zustände eine von zwei antrainierten äußeren Zielformen zugeordnet ist. So kann beispielsweise durch (jeweils vollständiges oder teilweises) Annehmen des ersten Zustandes und der ersten Zielform die Verbindung der Hohlelemente wie oben beschrieben hergestellt werden. Vorteilhafterweise kann durch Annehmen des zweiten Zustandes und der zweiten Zielform die beschriebene Verbindung wieder gelöst werden, d.h. durch ein entsprechendes Aktivieren des Formgedächtnisses des Formgedächtnismaterials und ohne Einbringen äußerer Kräfte.

Beispielsweise kann das Formgedächtnismaterial derart trainiert sein, dass das Formgedächtnismaterial bei Erwärmen auf oder über eine erste Aktivierungstemperatur in den ersten Zustand übergeht und bei Abkühlen auf oder unter eine zweite Aktivierungsenergie in den zweiten Zustand übergeht. In einem Ausführungsbeispiel entspricht die erste Aktivierungstemperatur etwa der menschlichen Körpertemperatur, also etwa 37° C. Die zweite Aktivierungstemperatur liegt dann vorzugsweise unterhalb der menschlichen Körpertemperatur, beispielsweise unter 0° C, wie etwa bei -100°C. Typischerweise sind die mittels des Zweiwegeffektes erzielbaren Kräfte geringer als die mittels des Einwegeffektes erzielbaren Kräfte. Außerdem sind Formgedächtnismaterialien, die ein Formgedächtnis mit Zweiwegeffekt aufweisen, typischerweise aufwendiger und damit teurer in der Herstellung als entsprechende Formgedächtnismaterialen mit Einwegeffekt. In der Regel sind aber die mit dem Zweiwegeffekt erzeugbaren Kräfte zur Herstellung einer stabilen Verbindung in einer implantierbaren Anordnung bereits ausreichend. Außerdem stellt im Fall von implantierbaren Anordnungen die relativ einfache Lösbarkeit der jeweiligen Verbindung mittels des Zweiwegeffektes oftmals einen besonders gewichtigen Vorteil dar. Entsprechend der obigen Beschreibung kann in der Ausgangsform (im Fall des Einwegeffektes) bzw. in der ersten Zielform (im Fall des Zweiwegeffektes) ein Innendurchmesser des jeweiligen Verbindungselementes kleiner sein als in der Zwischenform (im Fall des Einwegeffektes) bzw. in der zweiten Zielform (im Fall des Zweiwegeffektes). Alternativ hierzu kann in der Ausgangsform (im Fall des Einwegeffektes) bzw. in der ersten Zielform (im Fall des Zweiwegeffektes) ein Außendurchmesser des jeweiligen Verbindungselementes größer sein als in der Zwischenform (im Fall des Einwegeffektes) bzw. in der zweiten Zielform (im Fall des Zweiwegeffektes). Hierzu können die geometrische Ausgestaltung des Formgedächtnismaterials in den jeweiligen Zuständen und des Verbindungselements sowie der Hohlelemente entsprechend aufeinander abgestimmt sein, wie oben beschrieben worden ist.

Beispielsweise kann das Formgedächtnismaterial eine Formgedächtnislegierung sein oder beinhalten. Das Verbindungselement kann vollständig durch das Formgedächtnismaterial gegeben sein. Das Formgedächtnismaterial kann biokompatibel sein oder, wie weiter unten detaillierter beschrieben wird, mit einem biokompatiblen Material beschichtet, umhüllt oder umgossen sein, beispielsweise mit Silikon. Als Formgedächtnismaterial kommt insbesondere Nitinol in Frage.

Typischerweise sind das Formgedächtnismaterial und die Hohlelemente so aufeinander abgestimmt und/oder so weit voneinander beabstandet, dass durch das Erwärmen des Formgedächtnismaterials zum Aktivieren des Formgedächtnisses die Hohlelemente nicht beschädigt werden. Dies kann beispielsweise durch eine geeignete Auswahl des Formgedächtnismaterials und/oder der Materialien der Hohlelemente gewährleistet werden. Alternativ oder zusätzlich kann es bei der Aktivierung des Formgedächtnisses des Formgedächtnismaterials durch Erwärmen oder Abkühlen erforderlich sein, Maßnahmen zur Vorbeugung einer zu starken Erwärmung bzw. einer zu starken Abkühlung eines oder mehrerer der Hohlelemente vorzusehen. Beispielsweise kann eine Erwärmung des ersten Hohlelements und/oder des zweiten Hohlelements auf eine zulässige Maximaltemperatur (beispielsweise eine Schmelztemperatur des jeweiligen Materials) oder eine Abkühlung des ersten Hohlelements und/oder des zweiten Hohlelements auf eine zulässige Minimaltemperatur mittels einer thermischen Isolationsschicht begrenzt werden. Diese Isolationsschicht kann beispielsweise bereits vor dem Anordnen, Erwärmen bzw. Abkühlen des Formgedächtnismaterials zwischen (typischerweise radial zwischen) dem Formgedächtnismaterial bzw. dem Verbindungselement und dem ersten und/oder dem zweiten Hohlelement angeordnet werden. Beispielsweise kann diese thermische Isolationsschicht eine Hülse bzw. hülsenförmig sein. Die Isolationsschicht kann beispielsweise auf bzw. in eines dieser Hohlelemente axial auf- bzw. eingeschoben werden. Die Isolationsschicht kann auch ein axiales Endstück oder eine hülsenförmige axiale Auskragung (auch als Lippe bezeichnet) des ersten oder des zweiten Hohlelements sein. Dies kann besonders dann vorteilhaft sein, falls dieses Hohlelement beispielsweise aus einem weniger hitzeempfindlichen Material gefertigt ist als das jeweils andere Hohlelement, beispielsweise aus Silikon, während das andere Hohlelement aus einem wärmeempfindlicheren Material besteht, wie beispielsweise aus einem Polyester oder Polyestergewebe. Beispielsweise kann, wie im Folgenden näher beschrieben wird, das Formgedächtnismaterial in das Material (beispielsweise Silikon) eines der Hohlelemente eingebettet sein. Beispielsweise kann das Formgedächtnismaterial kann mit dem Material des Hohlelementes bzw. mit dem Material einer Isolationsschicht oder einer Zwischenschicht umhüllt oder umgossen sein.

Bereits vor dem Zusammensetzen des ersten Hohlelements und des zweiten Hohlelements kann mindestens ein Verbindungselement des mindestens einen Verbindungselements mit dem ersten Hohlelement oder mit dem zweiten Hohlelement fest verbunden werden bzw. sein. Alternativ oder zusätzlich kann auf entsprechende Weise und ebenfalls bereits vor dem Zusammensetzen des ersten Hohlelements und des zweiten Hohlelements das dritte Hohlelement, sofern ein solches vorgesehen ist, mit dem ersten Hohlelement oder mit dem zweiten Hohlelement fest verbunden werden bzw. sein. Dieses feste Verbinden des Verbindungselements, des Formgedächtnismaterials bzw. des dritten Hohlelements mit dem ersten bzw. zweiten Hohlelement kann jeweils beispielweise durch Einbetten in einen Teilbereich des ersten Hohlelements oder in einen Teilbereich des zweiten Hohlelements erfolgen. Der jeweilige Teilbereich kann ein axiales Endstück oder eine axiale Auskragung des jeweiligen Hohlelements sein. Das Einbetten kann durch ein Umgießen mit einem den Teilbereich bildendes Material bei der Herstellung dieses Teilbereichs erfolgen. Als Material kommt beispielsweise Silikon in Frage.

Das dritte Hohlelement, sofern vorhanden, kann eine Außenkontur aufweisen, die einen ersten axialen Bereich und einen zweiten axialen Bereich umfasst, wobei zwischen dem ersten axialen Bereich und dem zweiten axialen Bereich eine erste Stoßkante des dritten Hohlelements ausgebildet ist, welche den Fließkanal typischerweise in azimutaler Richtung umläuft. Beim Zusammensetzen der Anordnung wird vorzugsweise das dritte Hohlelements mit dem ersten axialen Bereich voran so weit in den ersten Hohlraum des ersten Hohlelements eingeschoben, bis eine Stirnseite des ersten Hohlelements an die erste Stoßkante anstößt. Beispielsweise kann ein Außendurchmesser des dritten Hohlelementes im ersten axialen Bereich kleiner sein als im zweiten axialen Bereich oder umgekehrt. Insbesondere kann der erste axiale Bereich direkt an den zweiten axialen Bereich angrenzen. Es ist außerdem möglich, dass das dritte Hohlelement zwischen dem ersten axialen Bereich und dem zweiten axialen Bereich einen Steg aufweist, der den Fließkanal azimutal umläuft und der auf einer Seite die erste Stoßkante ausbildet und auf einer entgegengesetzten weiteren Seite eine zweite Stoßkante ausbildet. Beim Zusammensetzen der Anordnung kann dann das dritte Hohlelement mit dem zweiten axialen Bereich voran so weit in den zweiten Hohlraum des zweiten Hohlelements eingeschoben werden, bis eine zweite Stirnseite des zweiten Hohlelements an die zweite Stoßkante anstößt.

Beispielsweise kann eine radiale Höhe der ersten Stoßkante kleiner sein als eine radiale Wanddicke bzw. radiale Höhe der Stirnseite des ersten Hohlelementes. Insbesondere kann die Stirnseite des ersten Hohlelements nach dem Einführen des dritten Hohlelements in den ersten Hohlraum des ersten Hohlelements radial nach außen über die Stoßkante hinausragen. Anschließend kann dann der zweite axiale Bereich des dritten Hohlelements so weit in den zweiten Hohlraum des zweiten Hohlelements eingeführt werden, bis eine Stirnseite des zweiten Hohlelements an die Stirnseite des ersten Hohlelements anstößt. Alternativ hierzu ist es möglich, dass zuerst das dritte Hohlelement mit dem zweiten axialen Bereich voran über die Stoßkante hinweg in den zweiten Hohlraum des zweiten Hohlelements eingeführt wird, so dass ein axiales Endstück des zweiten Hohlelements axial mit dem ersten axialen Bereich des dritten Hohlelements überlappt, wobei anschließend der erste axiale Bereich des dritten Hohlelements zusammen mit dem genannten axialen Endstück des zweiten Hohlelements in den ersten Hohlraum des ersten Hohlelements eingeführt wird, vorzugsweise möglichst bis zur Stoßkante. Das Verbindungselement kann anschließend axial überlappend mit dem ersten Hohlelement, dem genannten axialen Endstück des zweiten Hohlelements und dem ersten axialen Bereich des dritten Hohlelements angeordnet und in dieser Anordnung durch die Aktivierung des Formgedächtnismaterials wie beschrieben befestigt werden. Dann hintergreift das axiale Endstück des zweiten Hohlelements die Stoßkante, wodurch die Stabilität und Dichtheit der Anordnung gesteigert wird.

Wie oben bereits beschrieben worden ist, kann beispielsweise das erste Hohlelement oder auch das zweite Hohlelement eine axiale Auskragung bzw. Lippe aufweisen, welche beispielsweise als Isolierschicht oder als zusätzliche Zwischenschicht zur verbesserten Dichtung bzw. zum Ausgleichen von Unebenheiten dienen kann. Diese Auskragung kann axial über die oben genannte Stirnfläche des ersten Hohlelementes hinausragen. Nach dem Zusammensetzen des ersten Hohlelements mit dem zweiten Hohlelement und dem dritten Hohlelement kann diese Auskragung mit dem zweiten Hohlelement und mit dem oben genannten zweiten axialen Bereich des dritten Hohlelements axial überlappen. Alternativ oder zusätzlich zur beschriebenen Isolationsschicht und Auskragung können weitere, gleichartig ausgestaltete und entsprechend angeordnete Zwischenschichten vorgesehen sein, welche beispielsweise der verbesserten Dichtheit und/oder dem Ausgleich von Unebenheiten dienen.

Wie in der Einleitung der Beschreibung bereits dargestellt worden ist, kann das erste Hohlelement eine Kanüle, ein Schlauch, ein Graft (Gefäßprothese) oder ein Rohr sein. Ebenso kann das zweite Hohlelement eine Kanüle, ein Schlauch, eine Gefäßprothese (Graft) oder ein Rohr sein. Als Materialien für die Kanüle und den Schlauch kommt beispielsweise Silikon oder ein anderes biokompatibles Material in Frage. Für die Gefäßprothese (Graft) kommt beispielsweise eine Polyester bzw. ein Polyestergewebe in Betracht. Sofern die Anordnung auch das oben beschriebene dritte Hohlelement umfasst, ist das dritte Hohlelement typischerweise ein Rohr bzw. eine sogenannte Olive. Typischerweise ist eine Gefäßprothese (Graft) flexibler und nachgiebiger als ein Schlauch und eine Kanüle. Typischerweise ist außerdem ein Schlauch wie auch eine Kanüle flexibler und nachgiebiger als ein Rohr. Die weiter oben beschriebene mechanische (Gegen-)Spannung wird somit hauptsächlich von demjenigen Rohrelement mit der geringeren Flexibilität und Nachgiebigkeit erzeugt.

Das erste Hohlelement, das zweite Hohlelement und/oder das dritte Hohlelement (sofern vorhanden) kann bzw. können vollständig oder bereichsweise, vorzugsweise zumindest auf einer Oberfläche des ersten, zweiten bzw. dritten Hohlelements, aus einem biokompatiblen Kunststoff, insbesondere aus Silikon oder Polyester bzw. Polyestergewebe, oder aus einem (vorzugsweise ebenfalls biokompatiblen) metallischen Werkstoff, beispielsweise aus Titan, gefertigt sein. Weitere mögliche Materialien sind beispielsweise Edelstahl und Kobaltchromstahl. Insbesondere im Fall, dass der Fließkanal zum Leiten von Blut bestimmt ist, sind die Oberflächen der Innenwände der Hohlelemente, die also den ersten, zweiten bzw. dritten Hohlraum begrenzen, aus einem biokompatiblen Material gefertigt oder mit einem solchen Material beschichtet, wie beispielsweise einem der oben genannten Materialien. Außerdem können die Oberflächen des ersten, zweiten und/oder dritten Hohlelements zur Verbesserung der Biokompatibilität poliert sein, insbesondere wenn diese Oberflächen aus einem metallischen Werkstoff, wie etwa Titan, bestehen. Beispielsweise kann das erste Hohlelement eine Gefäßprothese sein. Beispielsweise kann das zweite Hohlelement (ebenfalls) eine Gefäßprothese sein. Beispielsweise kann das erste Hohlelement ein Silikonschlauch sein. Beispielsweise kann das zweite Hohlelement (ebenfalls) ein Silikonschlauch sein. Beispielsweise kann das erste Hohlelement eine Gefäßprothese und das zweite Hohlelement ein Silikonschlauch sein. Es können auch das erste Hohlelement und das zweite Hohlelement gleichzeitig eine Gefäßprothese oder gleichzeitig ein Silikonschlauch sein.

Vorteilhafterweise ist es möglich, das Verfahren wie eingangs beschrieben, vollständig oder teilweise, vor oder während einer Implantation der Anordnung in einen lebenden Körper durchzuführen. Insbesondere kann das Aktivieren des Formgedächtnisses des Formgedächtnismaterials des mindestens einen Verbindungselementes vor oder während der Implantation der Anordnung durchgeführt werden. Beispielsweise kann zunächst das zweite (oder erste) Hohlelement der Anordnung mit einer typischerweise bereits implantierten Blutpumpe verbunden werden, insbesondere wenn es sich bei diesem Hohlelement um einen Ein- oder Auslass der Blutpumpe handelt. Falls notwendig, kann dieses Hohlelement zunächst gekürzt werden, beispielsweise um es an gegebene anatomische Strukturen und vorliegende anatomische Größenverhältnisse anzupassen. In einem nachfolgenden Schritt wird es, wie oben beschrieben, mit dem ersten und/oder mit dem dritten Hohlelement verbunden.

Die hier vorgeschlagene implantierbare Anordnung wird vorzugsweise mittels des hier vorgeschlagenen Verfahrens hergestellt. Insofern kann die Beschreibung des hier vorgeschlagenen Verfahrens entsprechend auch auf die Anordnung übertragen werden. Die Anordnung umfasst demnach das oben beschriebene erste und zweite Hohlelement sowie das mindestens eine Verbindungselement, welches das erste Hohlelement mit dem zweiten Hohlelement verbindet. Das erste Hohlelement, das zweite Hohlelement sowie ggf. das dritte Hohlelement und das mindestens eine Verbindungselement sind dementsprechend so zusammengesetzt, dass der erste Hohlraum des ersten Hohlelements und der zweite Hohlraum des zweiten Hohlelements sowie ggf. der dritte Hohlraum des dritten Hohlelements einen Fließkanal für die Flüssigkeit ausbilden. Das mindestens eine Verbindungselement überlappt mit dem ersten Hohlelement und/oder mit dem zweiten Hohlelement sowie ggf. mit dem dritten Hohlelement axial. Das mindestens eine Verbindungselement besteht zumindest bereichsweise aus einem Formgedächtnismaterial, wie beispielsweise Nitinol. Das mindestens eine Verbindungselement verbindet die Hohlelemente vorzugsweise mittels eines Kraftschlusses und/oder mittels eines Formschlusses. Die hierfür erforderliche Kraft bzw. mechanische Spannung wird mittels des Formgedächtnismaterials erzeugt. Das Formgedächtnis ist hierzu aktiviert. Insbesondere kann das aktivierte Formgedächtnis unterdrückt sein, indem eine durch die Aktivierung des Formgedächtnis hervorgerufene Phasenumwandlung und die dadurch induzierte Verformung des Formgedächtnismaterials nur unvollständig bzw. bereichsweise vollzogen ist, insbesondere aufgrund einer von einem oder mehreren der genannten Hohlelemente hervorgerufenen und auf das Formgedächtnis einwirkenden mechanischen (Gegen-)Spannung, wie im Zusammenhang mit dem hier vorgeschlagenen Verfahren beschrieben worden ist. Zusätzlich bzw. alternativ hierzu kann das Formgedächtnismaterial ein trainiertes Formgedächtnis aufweisen. Beispielsweise ist es möglich, dass das Formgedächtnismaterial mittels einer entsprechenden Aktivierung seines trainierten Formgedächtnisses eigenständig, d.h. ohne Eintragen äußerer Kräfte, zwei verschiedene Zielformen annehmen kann, wie weiter oben beschreiben worden ist. Beispielsweise kann es so ermöglicht sein, dass die Verbindung durch eine entsprechende Aktivierung des Formgedächtnisses des Formgedächtnismaterials wieder lösbar ist.

Das hier vorgeschlagene Blutpumpensystem umfasst eine implantierbare Anordnung hier vorgeschlagener Art und eine mit dieser Anordnung verbundene oder verbindbare Blutpumpe zum Fördern von Blut durch den Fließkanal der Anordnung.

Im Folgenden werden das hier vorgeschlagene Verfahren, die hier vorgeschlagene implantierbare Anordnung und das hier vorgeschlagene Blutpumpensystem anhand der in den Figuren 1 bis 13 schematisch dargestellten speziellen Ausführungsbeispiele näher erläutert. Es zeigt:
- Figur 1: ein implantierbares Blutpumpensystem mit einer Anordnung hier vorgeschlagener Art zum Leiten von Blut und einer Blutpumpe,
- Figur 2: eine Teilansicht eines Querschnitts durch Hohlelemente der Anordnung des in Figur 1 gezeigten Blutpumpensystems, wobei die Schnittebene entlang einer Längsachse der Hohlelemente verläuft,
- Figur 3: die in Figur 2 gezeigten Hohlelemente mit Verbindungselementen der Anordnung,
- Figur 4: die in Figur 3 gezeigten Hohlelemente und Verbindungselemente nach einer Aktivierung eines Formgedächtnisses der Verbindungselemente,
- Figur 5A: die in Figuren 1, 3 und 4 gezeigten Verbindungselemente in einem Zustand A vor einer Aktivierung des Formgedächtnisses,
- Figur 5B: die in Figuren 1, 3 und 4 gezeigten Verbindungselemente in einem Zustand B nach einer Aktivierung des Formgedächtnisses,
- Fign. 6 bis 13: Weiterentwicklungen der in Figuren 1, 3 und 4 gezeigten Anordnung, wobei jeweils ein Teilausschnitt eines Querschnitts entlang der Längsachse der Hohlelemente gezeigt ist.

In den Figuren sind gleiche oder einander entsprechende Merkmale mit den gleichen Bezugszeichen gekennzeichnet.

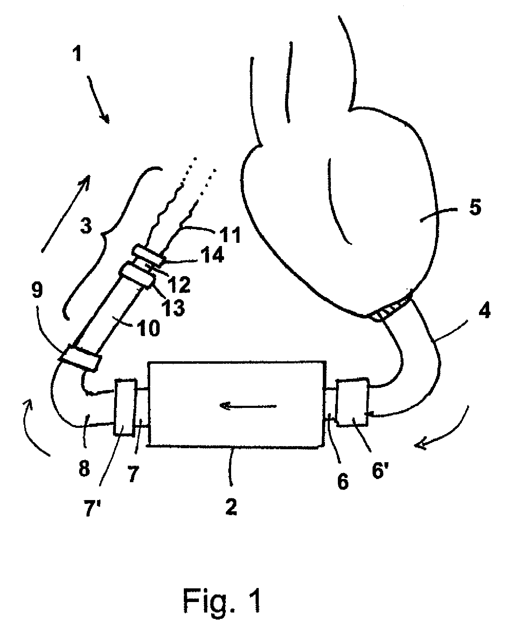

In Figur 1 ist ein in einen Körper eines lebenden Menschen implantiertes Blutpumpensystem 1 mit einer Blutpumpe 2 und einer Anordnung 3 hier vorgeschlagener Art schematisch dargestellt. Eine Einlasskanüle 4 ist mit einem linken Ventrikel (hier nicht dargestellt) eines Herzens 5 verbunden und leitet Blut durch einen Einlass 6, der ein Rohrstück mit einem Kupplungselement 6' ist, in die Blutpumpe 2 (der Strömungsverlauf des Bluts durch das Blutpumpensystem 1 ist durch Pfeile dargestellt), in der das Blut mittels eines motorisierten Pumpenrads angetrieben und durch einen Auslass 7, welcher ebenfalls ein Rohrstück mit einem Kupplungselement 7' ist, in einen Auslasskrümmer 8 (ein gekrümmtes Rohrstück) gefördert wird. Dieser Auslasskrümmer 8 ist über ein Kupplungselement 9 mit der Anordnung 3 verbunden, so dass das Blut in diese Anordnung 3 hinein und durch sie hindurch geleitet wird.

Die Anordnung 3 ist mit einem Verfahren hier vorgeschlagener Arte hergestellt worden und umfasst ein biokompatibles erstes Hohlelement 10, ein biokompatibles zweites Hohlelement 11 und ein biokompatibles drittes Hohlelement 12 sowie zwei biokompatible Verbindungselemente 13, 14, welche das erste Hohlelement 10, das zweite Hohlelement 11 und das dritte Hohlelement 12 miteinander verbinden. In einem alternativen Blutpumpensystem 1 kann die Anordnung 3 direkt mit dem Auslass 7 verbunden sein. Dann entspricht der Auslass 7 beispielsweise dem ersten Hohlelement 10 und das Kupplungselement 7 beispielsweise dem Verbindungselement 13. Es kann die Anordnung 3 prinzipiell auch mit dem Einlass 6 verbunden sein. Dann entspricht beispielsweise der Einlass 6 dem ersten Hohlelement 10 und das Kupplungselement 6' beispielsweise dem Verbindungselement 13.

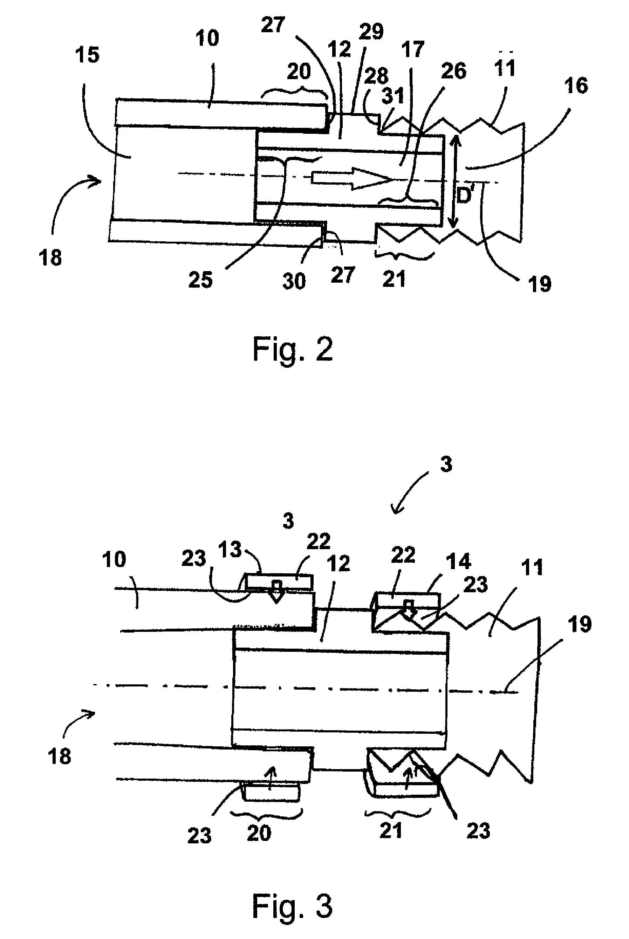

Im hier gezeigten Beispiel ist das erste Hohlelement 10 ein relativ biegeweicher Schlauch aus Silikon, könnte aber auch ein relativ biegesteifes Rohr sein. Das zweite Hohlelement 11 ist eine biegeweiche Gefäßprothese (Graft) aus einem Polyestergewebe. Das dritte Hohlelement 12 ist eine Olive aus möglichst fein poliertem Titan. Bei den beiden Verbindungselementen 13, 14 handelt es sich jeweils um eine hohlzylindrische Hülse, die also jeweils eine kreisringförmige (rotationssymmetrische) Querschnittsfläche 24 aufweist, siehe Figuren 3A und 3B. Die Verbindungselemente 13, 14 sind in diesem Beispiel vollständig aus einem Formgedächtnismaterial gefertigt, welches im gezeigten Beispiel durch eine Formgedächtnislegierung gegeben ist, in diesem speziellen Fall durch Nitinol.

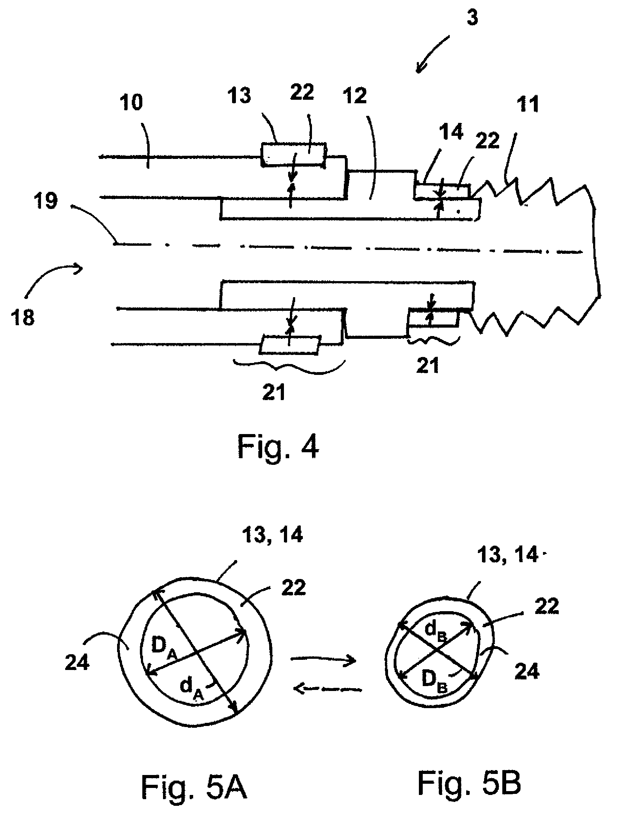

Wie in Figur 2 gezeigt ist, werden das erste Hohlelement 10, das zweite Hohlelement 11 und das dritte Hohlelement 12 der in Figur 1 gezeigten Anordnung 3 zunächst so zusammengesetzt, dass ein erster Hohlraum 15 des ersten Hohlelements 10, ein zweiter Hohlraum 16 des zweiten Hohlelements 11 und ein dritter Hohlraum 17 des dritten Hohlelements 12 einen durchgängigen Fließkanal 18 für das Blut ausbilden. In Figuren 2 und 4 ist eine Längsachse 19 (gestrichelte Linie) des Fließkanals 18 eingezeichnet. Somit beziehen sich die im Folgenden verwendeten Begriffe axial, radial und azimutal auf diese Längsachse 19. Wie in Figur 2 gezeigt ist, überlappen das erste Hohlelement 10 und das dritte Hohlelement 12 in einem ersten axialen Überlappungsbereich 20 und überlappen das dritte Hohlelement 12 und das zweite Hohlelement in einem zweiten axialen Überlappungsbereich 21. Das Zusammensetzen kann beispielsweise vor oder auch während einer Implantation des Blutpumpensystems 1 erfolgen, beispielsweise nachdem das erste Hohlelement 10 mit der bereits implantierten Blutpumpe 2 verbunden und anschließend ggf. gekürzt worden ist.

Das dritte Hohlelements 12 weist einen ersten axialen Bereich 25 und einen zweiten axialen Bereich 26 auf, wobei axial zwischen dem ersten axialen Bereich 25 und dem zweiten axialen Bereich 26 eine erste Stoßkante 27 und eine zweite Stoßkante 28 sowie ein durch diese Stoßkanten gebildeter, radial nach außen weisender Steg 29 angeordnet ist, welche jeweils den Fließkanal 18 in azimutaler Richtung umlaufen. Beim Zusammensetzen der Anordnung 3 wird das dritte Hohlelement 12 mit dem ersten axialen Bereich 25 voran so weit in den ersten Hohlraum 15 des ersten Hohlelements 10 eingeschoben, bis eine Stirnseite 30 des ersten Hohlelements 10 an die erste Stoßkante 27 anstößt. Entsprechend wird das dritte Hohlelement 12 mit dem zweiten axialen Bereich 26 voran so weit in den zweiten Hohlraum 17 des zweiten Hohlelements 11 eingeschoben, bis eine Stirnseite 31 des zweiten Hohlelements 11 an die zweite Stoßkante 28 anstößt. Wie außerdem zu erkennen ist, ist eine radiale Höhe der ersten Stoßkante 27 kleiner als eine radiale Wanddicke bzw. radiale Höhe der Stirnseite 30 des ersten Hohlelementes 10, so dass die Stirnseite 30 des ersten Hohlelements 10 nach dem Einführen des dritten Hohlelements 12 in den ersten Hohlraum 15 des ersten Hohlelements 10 radial nach außen über die Stoßkante 30 hinausragt.

Das erste Hohlelement 10 und das zweite Hohlelement 11 erstrecken sich in axialer Richtung größtenteils außerhalb der Überlappungsbereiche 20, 21, da die axialen Gesamtlängen des ersten Hohlelements 10 und des zweiten Hohlelements 11 wesentlich größer als die axiale Ausdehnung der Überlappungsbereiche 20, 21 sind, beispielsweise um mehr als das Zehnfache. Dementsprechend sind in den Figuren 1 bis 4 und 6 bis 13 jeweils nur axiale Teilabschnitte des ersten und zweiten Hohlelements 10, 11 gezeigt. Das dritte Hohlelement 12 erstreckt sich in axialer Richtung zu einem großen Teil innerhalb der Überlappungsbereiche 20, 21, wird also zu einem großen Teil von dem ersten und dem zweiten Hohlelement 10, 11 axial überlappt, so dass die axiale Gesamtlänge des dritten Hohlelements 12 beispielsweise zu mindestens 80 % auf diese Weise überlappt ist.

Wie in Figur 3 zu sehen ist, werden anschließend die beiden Verbindungselemente 13, 14 (ggf. nach einem "Auffädeln" über die Hohlelemente 10, 11, 12) durch manuelles axiales Verschieben über die Hohlelemente 10, 11, 12 hinweg jeweils vollständig in den ersten bzw. in den zweiten axialen Überlappungsbereich 20, 21 hinein bewegt, so dass das eine Verbindungselement 13 mit dem ersten Hohlelement 10 und dem dritten Hohlelement 12 axial überlappt und das andere Verbindungselement 14 mit dem zweiten und dritten Hohlelement 11, 12 axial überlappt. Außerdem umlaufen die Verbindungselemente 13, 14 den Fließkanal azimutal und sind radial außerhalb der Hohlelemente 10, 11, 12 angeordnet. In einer alternativen Ausführungsform sind sie radial innerhalb der Hohlelemente 10, 11, 12 angeordnet (dann mit einer entgegengesetzten Kraft bzw. Presswirkung, siehe unten).

Nach dem (axialen) Zusammensetzen der Hohlelemente 10, 11, 12 und Verbindungselemente 13, 14 wird ein Formgedächtnis des Formgedächtnismaterials 22 der Verbindungselemente 13, 14 aktiviert, wodurch ein Phasenübergang (Phasenumwandlung) des Formgedächtnismaterials 22 und eine damit einhergehende Verformung des Formgedächtnismaterials 22 ausgelöst wird. Diese Verformung ist durch Figuren 5A und 5B stark schematisiert dargestellt, wobei in Figur 5A der Zustand bzw. die Form der Verbindungselemente 13, 14 vor der Verformung und in Figur 5B der Zustand bzw. die Form der Verbindungselemente 13, 14 nach der Verformung dargestellt ist. Durch die Verformung haben sich die hohlzylindrischen Verbindungselemente 13, 14 radial verengt, so dass sich jeweils ein Innendurchmesser d wie auch ein Außendurchmesser D der Verbindungselemente 13, 14 verkleinert hat. Der Index A bzw. B dient der Unterscheidung des jeweils vorliegenden Zustands des Formgedächtnismaterials, wobei Zustand A, gezeigt in Figur 5A, vor der Aktivierung vorliegt und Zustand B, gezeigt in Figur 5B, nach der Aktivierung. Es gilt d_{A} > d_{B} und D_{A} > D_{B}.

Durch diese Verformung ziehen sich die Verbindungselemente 13, 14 praktisch exakt radial zusammen, so dass sich die Innendurchmesser der Verbindungselemente 13, 14 jeweils dem Außendurchmesser D' des dritten Hohlelementes 12, siehe Figur 2, annähern. Hierdurch werden die in Figur 3 sichtbaren Zwischenräume 23 zwischen den Verbindungselementen 13, 14 und den Hohlelementen 10, 11 geschlossen und ein dauerhafter, flächiger Berührungskontakt zwischen den Verbindungselementen 13, 14 und dem ersten Hohlelement 10 bzw. dem zweiten Hohlelement 11 hergestellt. Über diesen Berührungskontakt übertragen die Verbindungselemente 13, 14 die durch die Aktivierung hervorgerufene mechanische Spannung des Formgedächtnismaterials 22 auf die Hohlelemente 10, 11, 12 und rufen in den jeweiligen Hohlelementen 10, 11, 12 eine entgegengesetzte mechanische Spannung hervor, siehe die einander entgegengesetzten radialen Pfeile in Figur 4. Die Verformung setzt sich so weit fort, bis die durch die Hohlelemente erzeugte mechanische (Gegen-) Spannung (ausgehend hauptsächlich von dem in diesem Ausführungsbeispiel stabilsten dritten Hohlelement 12) die von dem Formgedächtnismaterial erzeugte Spannung kompensiert und eine weitere Verformung des Verbindungselements verhindert wird. (Da radial zwischen dem starren dritten Hohlelement 12 und den Verbindungselementen 13, 14 das erste bzw. zweite Hohlelement 10, 11 angeordnet ist, gilt am Ende jeweils d_{B} > D'). In diesem Zustand, der prinzipiell unbegrenzt lange, also insbesondere Tage, Wochen, Monate oder Jahre anhalten kann, pressen die Verbindungselemente 13, 14 die Hohlelemente 10, 11, 12 aufeinander, so dass diese fest (kraftschlüssig) miteinander verbunden sind. Aufgrund der hohen Anpresskräfte durch das Formgedächtnismaterial 22 kann eine besonders zuverlässige Dichtigkeit erzielt werden. Dies wird dadurch noch gesteigert, dass bei der Herstellung der Verbindung eine (in der Regel unerwünschte) Faltenbildung des relativ dünnen Materials (Polyestergewebe) des zweiten Hohlelements 11 (Gefäßprothese, Graft) minimiert oder sogar ganz vermieden werden kann, da sich das Formgedächtnismaterial 22 der rotationssymmetrischen (hohlzylindrischen) Verbindungselemente 13, 14 praktisch exakt radial zusammenzieht. Derartige Falten könnten unter Umständen zu Undichtigkeiten führen, insbesondere bei Verwendung der hier allgemein vorgeschlagenen Anordnung zur Leitung von Körperflüssigkeiten, die von Blut verschieden sind bzw. die nicht gerinnend sind, wie beispielsweise Urin oder Gallenflüssigkeit.

Die durch die Aktivierung des Formgedächtnisses des Formgedächtnismaterials 22 ausgelöste Phasenumwandlung des Formgedächtnismaterials 22 (von Zustand A nach Zustand B, siehe Figuren 5A und 5B) kann aufgrund der beschriebenen Gegenspannung des dritten Hohlelements 12 beendet werden und auf diese Weise unvollständig bleiben (es können somit im Formgedächtnismaterial zwei Phasen gleichzeitig bestehen bleiben). Beispielsweise kann das Formgedächtnis nach seiner Aktivierung aufgrund der Gegenspannung des dritten Hohlelementes 12 "unterdrückt" sein.

Im vorliegenden Beispiel kann, je nach Wahl des Formgedächtnismaterials, entweder der sogenannte Einwegeffekt oder der sogenannte Zweiwegeffekt von Formgedächtnismaterialien ausgenutzt werden. Bei der Phasenumwandlung kann es sich um eine "martensitische Transformation" handeln, wie sie weiter oben beschrieben worden ist. Dann kann in dem in Figur 5A gezeigten Zustand A das Formgedächtnismaterial 22 beispielsweise in der "entzwillingten" martensitischen Phase vorliegen und in dem in Figur 5B gezeigten Zustand B in der austenitischen Phase vorliegen. Es ist auch möglich, dass das Formgedächtnismaterial 22 im Zustand A einen größeren Anteil der "entzwillingten" martensitischen Phase aufweist als in dem Zustand B und in dem Zustand A einen kleineren Anteil der austenitischen Phase aufweist als in dem Zustand B, insbesondere wenn das Formgedächtnis im Zustand B unterdrückt ist. Die Aktivierung kann, wie oben beschrieben worden ist, beispielsweise durch Erwärmen des Formgedächtnismaterials ausgelöst werden, beispielsweise durch Erwärmen über die sogenannte Austenit-Start-Temperatur (Aₛ) oder über eine Austenit-End-Temperatur (Aₑ) des Formgedächtnismaterials.

In einer alternativen Ausführungsform wird das Formgedächtnis des Formgedächtnismaterials 22 (vor dem Zusammensetzen der Verbindungselemente 13, 14 mit den Hohlelementen 10, 11, 12) trainiert und der "Zweiwegeffekt" des Formgedächtnismaterials 22 ausgenutzt, wobei sich das Formgedächtnis zwischen den beiden Zuständen bzw. Zielformen A und B in Figuren 5A und 5B durch entsprechende Aktivierungen des Formgedächtnisses hin und her (durchgezogener Pfeil bzw. gestrichelter Pfeil zwischen Figuren 5A und 5B) transformieren lässt.

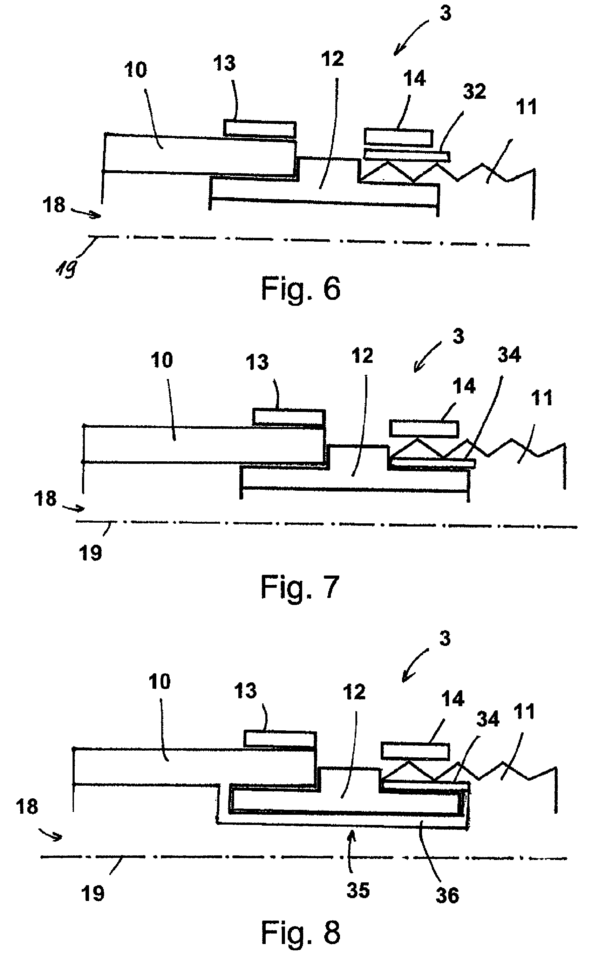

Die Figuren 6 bis 13 zeigen Teilansichten auf Querschnitte von Weiterentwicklungen der in Figuren 1, 3 und 4 gezeigten Anordnung 3, wobei die Schnittebene jeweils entlang der Längsachse 19 verläuft. Gezeigt sind der Übersichtlichkeit halber jeweils nur diejenigen Teile der Anordnung 3, die jeweils oberhalb der Längsachse 19 angeordnet sind. Gezeigt ist außerdem jeweils ein Zustand der Anordnung vor der Aktivierung des Formgedächtnismaterials. Beschrieben sind jeweils die Unterschiede von der in Figuren 1, 3 und 4 gezeigten Anordnung 3. Die im Folgenden beschriebenen Weiterentwicklungen können auch miteinander kombiniert werden, um mehrere der beschriebenen Vorteile gleichzeitig zu realisieren.

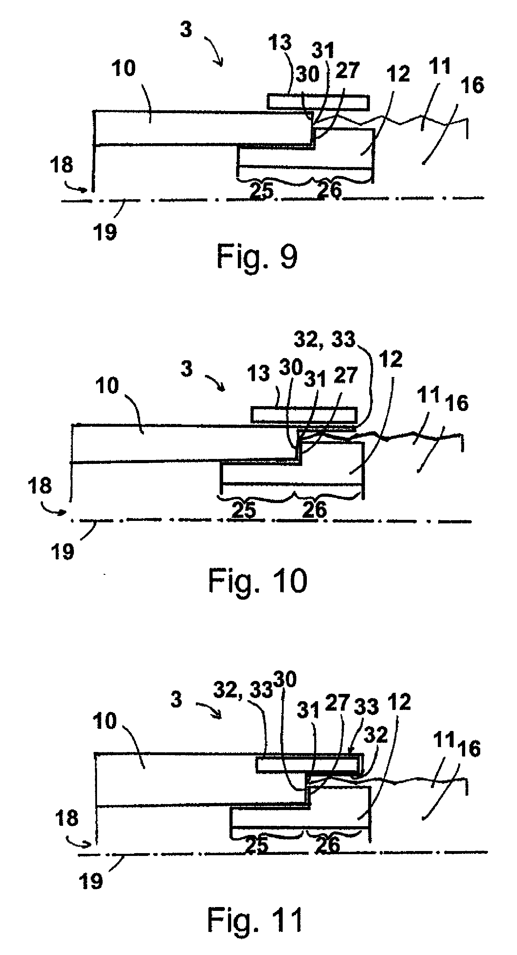

In den in Figuren 6 und 10 gezeigten Weiterentwicklungen umfasst die Anordnung eine thermische Isolierschicht 32, um einer zu starken Erwärmung (bzw. einer zu starken Abkühlung) des zweiten Hohlelementes 11 beim Aktivieren des Formgedächtnismaterials 22 des Verbindungselements 13 vorzubeugen. Diese Isolationsschicht wird bereits vor dem Anordnen des Verbindungselements 13 angeordnet, so dass es vor der Aktivierung des Formgedächtnismaterials radial zwischen dem Formgedächtnismaterial und dem zweiten Hohlelement 11 angeordnet ist. Die thermische Isolationsschicht 32 ist in dem in Figur 6 gezeigten Beispiel eine eigenständige Hülse aus Silikon, die unabhängig von dem ersten Hohlelement 10 auf das zweite Hohlelement 11 axial aufgeschoben wird. In dem in Figur 10 gezeigten Beispiel ist die Isolationsschicht 32 eine hülsenförmige axiale Auskragung 33 des ersten Hohlelements 10, welches, wie oben beschrieben worden ist, aus Silikon gefertigt ist. Wie beispielsweise in Figuren 11 gezeigt ist, kann das Formgedächtnismaterial 22 bzw. das Verbindungselement 13 in das Material (in diesem Beispiel Silikon) des ersten Hohlelementes 10 eingebettet sein, indem es beispielsweise mit diesem Material umgossen ist oder von diesem Material umhüllt ist. Beispielsweise ist das Formgedächtnismaterial 22 direkt in die genannte Auskragung 33 des ersten Hohlelements 10 eingebettet, wie in Figur 11 gezeigt ist.

Wie insbesondere in Figuren 7, 8 und 12 gezeigt ist, ist es prinzipiell möglich, radial zwischen den Hohlelementen, beispielsweise zwischen dem zweiten und dem dritten Hohlelement 11, 12, eine oder mehrere Zwischenschichten 34 anzuordnen, beispielsweise um Unebenheiten auszugleichen, um eine bessere Abdichtung zu bewirken und/oder um empfindliche Materialien vor einer mechanischen Überbeanspruchung, beispielsweise durch die von den Verbindungselementen hervorgerufenen mechanischen Spannungen, zu schützen. Die Zwischenschichten 34 sind hierzu typischerweise aus einem nachgiebigen und vorzugsweise ebenfalls biokompatiblen Material, wie etwa Silikon, gefertigt.

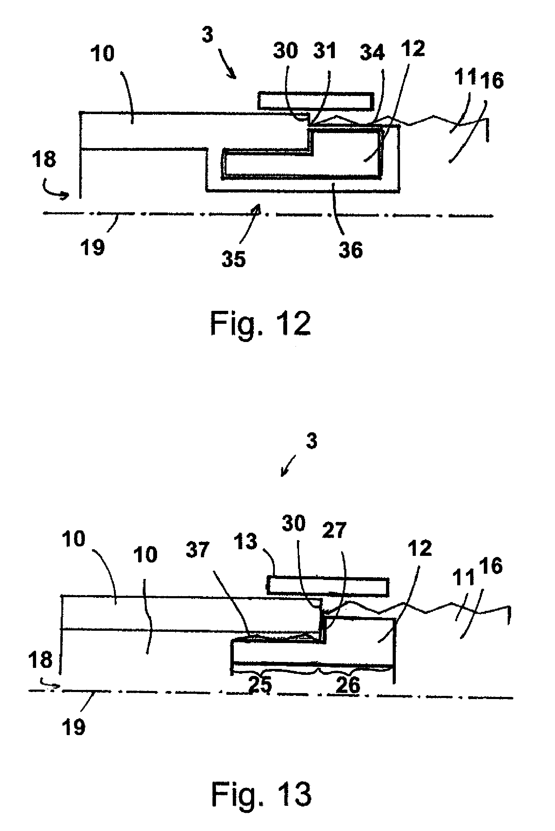

Wie in Figuren 8 und 12 gezeigt ist, kann beispielsweise bereits vor dem Zusammensetzen des ersten Hohlelements 10 und des zweiten Hohlelements 11 das dritte Hohlelement 12 mit dem ersten Hohlelement 10 fest verbunden werden, beispielweise durch Einbetten des dritten Hohlelements 12 in ein axiales Endstück 35 des ersten Hohlelements 11. Das Einbetten kann im vorliegenden Beispiel durch Umgießen des dritten Hohlelements 12 mit Silikon erfolgen. Hierdurch wird das Zusammensetzen der Hohlelemente vor oder während der Implantation vereinfacht. Außerdem werden hierdurch sowohl die Zwischenschicht 34 wie außerdem auch eine innenseitige Beschichtung 36 des dritten Hohlelements 36 mit Silikon gebildet. Auf diese Weise hat das Blut beim Durchfließen der Anordnung 3 ausschließlich Kontakt zu nur einem einzigen Werkstoff (Silikon), was erfahrungsgemäß blutschonend ist.

In den in Figuren 9 bis 13 gezeigten Weiterentwicklungen grenzt der erste axiale Bereich 25 des dritten Hohlelements 12 direkt an den zweiten axialen Bereich 26 des dritten Hohlelements 12 an. Wie in Figuren 9 bis 12 gezeigt ist, kann somit beim Zusammensetzen der Anordnung 3 der zweite axiale Bereich 26 des dritten Hohlelements 12 so weit in den zweiten Hohlraum 16 des zweiten Hohlelements 11 eingeführt werden, bis die Stirnseite 31 des zweiten Hohlelements 11 an die Stirnseite 30 des ersten Hohlelements 10 anstößt. Wie alternativ in Figur 13 gezeigt ist, kann auch zuerst das dritte Hohlelement 12 mit dem zweiten axialen Bereich 26 voran über die Stoßkante 27 hinweg in den zweiten Hohlraum 16 des zweiten Hohlelements 11 eingeführt werden, so dass ein axiales Endstück 37 des zweiten Hohlelements 11 axial mit dem ersten axialen Bereich 25 des dritten Hohlelements 12 überlappt. Anschließend wird dann der erste axiale Bereich 25 des dritten Hohlelements 12 zusammen mit diesem axialen Endstück 37 in den ersten Hohlraum 15 des ersten Hohlelements 10 bis zur Stoßkante 27 eingeführt. Das Verbindungselement wird anschließend axial überlappend mit dem ersten Hohlelement 10, dem Endstück 37 des zweiten Hohlelements 11 und dem ersten axialen Bereich 25 des dritten Hohlelements 12 angeordnet und in dieser Anordnung durch die Aktivierung des Formgedächtnismaterials wie beschrieben befestigt. Auf diese Weise hintergreift das axiale Endstück 37 des zweiten Hohlelements 11 die Stoßkante 27 und steigert so die Stabilität und Dichtheit der Anordnung 3 noch weiter.

Es wird, insbesondere auch mit Bezug auf Figuren 1 bis 8, darauf hingewiesen, dass das erste und das dritte Hohlelement 10, 12 zusammen mit dem Verbindungselement 13 bereits eine vollständige Anordnung hier vorgeschlagener Art bilden können, ebenso das zweite und das dritte Hohlelement 11, 12 zusammen mit dem Verbindungselement 14. Prinzipiell ist es daher möglich, das jeweils andere Verbindungselement der Anordnung ohne Formgedächtnismaterial auszuführen, beispielsweise als Crimpring, als C-Schelle oder als Fadenwicklung.

### Bezugszeichenliste:

- 1: Blutpumpensystem
- 2: Blutpumpe
- 3: Anordnung
- 4: Einlasskanüle
- 5: Herz
- 6: Einlass
- 6': Kupplungselement
- 7: Auslass
- 7': Kupplungselement
- 8: Auslasskrümmer
- 9: Kupplungselement
- 10: erstes Hohlelement
- 11: zweites Hohlelement
- 12: drittes Hohlelement
- 13: Verbindungselement
- 14: Verbindungselement
- 15: erster Hohlraum
- 16: zweiter Hohlraum
- 17: dritter Hohlraum
- 18: Fließkanal
- 19: Längsachse
- 20: Überlappungsbereich
- 21: Überlappungsbereich
- 22: Formgedächtnismaterial
- 23: Zwischenraum
- 24: Querschnittsfläche
- 25: axialer Bereich
- 26: axialer Bereich
- 27: Stoßkante
- 28: Stoßkante
- 29: Steg
- 30: Stirnseite
- 31: Stirnseite
- 32: Isolierschicht
- 33: Auskragung
- 34: Zwischenschicht
- 35: Endstück
- 36: Beschichtung
- 37: Endstück

## Patentansprüche

1. Verfahren zum Herstellen einer implantierbaren Anordnung (3) zum Leiten einer Flüssigkeit, insbesondere von Blut, bei dem
- ein biokompatibles oder biokompatibel umhülltes erstes Hohlelement (10) und ein biokompatibles oder biokompatibel umhülltes zweites Hohlelement (11) sowie mindestens ein Verbindungselement (13, 14) zum Verbinden des ersten Hohlelements (10) mit dem zweiten Hohlelement (11) bereitgestellt werden, wobei das mindestens eine Verbindungselement (13, 14) zumindest bereichsweise aus einem Formgedächtnismaterial (22) besteht,
- wobei das erste Hohlelement (10), das zweite Hohlelement (11) und das mindestens eine Verbindungselement (13, 14) so zusammengesetzt werden, dass ein erster Hohlraum des ersten Hohlelements (10) und ein zweiter Hohlraum des zweiten Hohlelements (11) einen Fließkanal (18) für die Flüssigkeit ausbilden und das mindestens eine Verbindungselement (13, 14) mit dem ersten Hohlelement (10) und/oder mit dem zweiten Hohlelement (11) axial überlappt,
- wobei anschließend ein Formgedächtnis des Formgedächtnismaterials (22) des mindestens einen Verbindungselementes (13, 14) aktiviert wird, so dass in dem Formgedächtnismaterial (22) eine mechanische Spannung erzeugt wird und sich das Formgedächtnismaterial (22) des mindestens einen Verbindungselements (13, 14) verformt, wobei das mindestens eine Verbindungselement (13, 14) zumindest einen Teil der mechanischen Spannung des Formgedächtnismaterials (22) auf das erste Hohlelement (10) und/oder auf das zweite Hohlelement (11) überträgt.

2. Verfahren nach Anspruch 1, **dadurch gekennzeichnet, dass** das erste Hohlelement (10) und das zweite Hohlelement (11) so zusammengesetzt werden, dass sie sich in einem Überlappungsbereich (20, 21) axial überlappen, wobei das mindestens eine Verbindungselement (13, 14) in diesem Überlappungsbereich (20, 21) angeordnet wird, wobei das erste Hohlelement (10) und das zweite Hohlelement (11) durch das mindestens eine Verbindungselement (13, 14) nach dem Aktivieren des Formgedächtnisses aufeinandergepresst werden.

3. Verfahren nach einem der vorangehenden Ansprüche, **dadurch gekennzeichnet, dass** ein drittes Hohlelement bereitgestellt wird, wobei das erste Hohlelement (10), das zweite Hohlelement (11) und das dritte Hohlelement (12) so zusammengesetzt werden, dass ein dritter Hohlraum des dritten Hohlelements mit dem ersten Hohlraum des ersten Hohlelements (10) und dem zweiten Hohlraum des zweiten Hohlelements (11) den genannten Fließkanal (18) ausbildet und dass das dritte Hohlelement (12) sowohl mit dem ersten Hohlelement als auch mit dem zweiten Hohlelement in mindestens einem Überlappungsbereich (20, 21) axial überlappt, wobei das mindestens eine Verbindungselement (13, 14) in diesem mindestens einen Überlappungsbereich (20, 21) angeordnet wird, wobei, nach dem Aktivieren des Formgedächtnismaterials (22) des mindestens einen Verbindungselements (13, 14), durch das mindestens eine Verbindungselement (13, 14) das erste Hohlelement (10) und das dritte Hohlelement (12) aufeinandergepresst und auch das zweite Hohlelement (11) und das dritte Hohlelement (12) aufeinandergepresst werden.

4. Verfahren nach einem der vorangehenden Ansprüche, **dadurch gekennzeichnet, dass** das Formgedächtnismaterial (22) des mindestens einen Verbindungselements (13, 14) mindestens einen Ring, der vorzugsweise ringsum geschlossen ist, oder mindestens eine Hülse, die vorzugsweise ringsum geschlossen ist, bildet, wobei der mindestens eine Ring bzw. die mindestens eine Hülse den Fließkanal (18) umläuft.

5. Verfahren nach einem der vorangehenden Ansprüche, **dadurch gekennzeichnet, dass** das Formgedächtnismaterial eine Formgedächtnislegierung ist oder beinhaltet, wie beispielsweise Nitinol.

6. Verfahren nach einem der vorangehenden Ansprüche, **dadurch gekennzeichnet, dass** nach dem Aktivieren des Formgedächtnisses des Formgedächtnismaterials (22) eine atomare Gitterstruktur des Formgedächtnismaterials (22) zumindest bereichsweise von einer martensitischen Gitterstruktur in eine austenitische Gitterstruktur übergeht.

7. Verfahren nach einem der vorangehenden Ansprüche, **dadurch gekennzeichnet, dass** das Formgedächtnis des Formgedächtnismaterials (22) des mindestens einen Verbindungselements (13, 14) vor dem Zusammensetzen des mindestens einen Verbindungselements (13, 14) mit dem ersten Hohlelement (10) und mit dem zweiten Hohlelement in mehreren thermomechanischen Behandlungszyklen trainiert wird.

8. Verfahren nach einem der vorangehenden Ansprüche, **dadurch gekennzeichnet, dass** das Formgedächtnis des Formgedächtnismaterials (22) des mindestens einen Verbindungselements (13, 14) durch Erwärmen des Formgedächtnismaterials (22) aktiviert wird, beispielsweise über eine Austenit-Start-Temperatur (Aₛ) oder über eine Austenit-End-Temperatur (Aₑ) des Formgedächtnismaterials.

9. Verfahren nach Anspruch 8, **dadurch gekennzeichnet, dass** eine Erwärmung oder Abkühlung des ersten Hohlelements (10) und/oder des zweiten Hohlelements (11) auf eine zulässige Maximaltemperatur oder Minimaltemperatur mittels einer thermischen Isolationsschicht, die zwischen dem Formgedächtnismaterial (22) und dem ersten und/oder dem zweiten Hohlelement angeordnet ist, begrenzt wird, wobei diese thermische Isolationsschicht vorzugsweise eine Hülse oder ein axiales Endstück oder eine hülsenförmige axiale Auskragung des ersten oder des zweiten Hohlelements (11) ist.

10. Verfahren nach einem der vorangehenden Ansprüche, **dadurch gekennzeichnet, dass** zunächst das erste Hohlelement (10) und das zweite Hohlelement (11) relativ zueinander bis zu einer Endausrichtung ausgerichtet werden und anschließend das mindestens eine Verbindungselement (13, 14) relativ zum ersten Hohlelement und relativ zum zweiten Hohlelement bis zu einer axialen Endposition des mindestens einen Verbindungselements (13, 14) axial verschoben wird, insbesondere durch manuelles Verschieben des mindestens einen Verbindungselements (13, 14) relativ zum ersten Hohlelement und relativ zum zweiten Hohlelement.

11. Verfahren nach einem der Ansprüche 1 bis 9, **dadurch gekennzeichnet, dass** bereits vor dem Zusammensetzen des ersten Hohlelements (10) und des zweiten Hohlelements (11) mindestens ein Verbindungselement (13, 14) des mindestens einen Verbindungselements (13, 14) mit dem ersten Hohlelement (10) oder mit dem zweiten Hohlelement (11) fest verbunden wird, vorzugsweise durch Einbetten des Formgedächtnismaterials (22) des mindestens einen Verbindungselements (13, 14) in einen Teilbereich des ersten Hohlelements (10) oder in einen Teilbereich des zweiten Hohlelements, oder dass, sofern dieser Anspruch mit Anspruch 3 kombiniert wird, bereits vor dem Zusammensetzen des ersten Hohlelements (10) und des zweiten Hohlelements (11) das dritte Hohlelement (12) mit dem ersten Hohlelement (10) oder mit dem zweiten Hohlelement (11) fest verbunden wird, vorzugsweise durch Einbetten des dritten Hohlelements in einen Teilbereich des ersten Hohlelements (10) oder in einen Teilbereich des zweiten Hohlelements.

12. Verfahren nach einem der vorangehenden Ansprüche, **dadurch gekennzeichnet, dass** das erste Hohlelement (10) zumindest bereichsweise aus einem biokompatiblen Kunststoff, insbesondere aus Silikon oder Polyester, oder einem metallischen Werkstoff, insbesondere Titan, gefertigt ist und/oder dass das zweite Hohlelement (11) zumindest bereichsweise aus einem biokompatiblen Kunststoff, insbesondere aus Silikon oder Polyester, oder einem metallischen Werkstoff, insbesondere Titan, gefertigt ist und/oder, soweit dieser Anspruch mit Anspruch 3 kombiniert wird, dass das dritte Hohlelement (12) aus einem metallischen Werkstoff, insbesondere aus Titan, gefertigt ist.

13. Verfahren nach einem der vorangehenden Ansprüche, **dadurch gekennzeichnet, dass** das Aktivieren des Formgedächtnisses des Formgedächtnismaterials (22) des mindestens einen Verbindungselementes (13, 14) vor oder während einer Implantation der Anordnung (3) durchgeführt wird.

14. Implantierbare Anordnung (3) zum Leiten einer Flüssigkeit, insbesondere von Blut, umfassend ein biokompatibles oder biokompatibel umhülltes erstes Hohlelement (10) und ein biokompatibles oder biokompatibel umhülltes zweites Hohlelement (11) sowie mindestens ein Verbindungselement (13, 14) zum Verbinden des ersten Hohlelements (10) mit dem zweiten Hohlelement (11), wobei das mindestens eine Verbindungselement zumindest bereichsweise aus einem Formgedächtnismaterial (22) besteht (13, 14), wobei das erste Hohlelement (10), das zweite Hohlelement (11) und das mindestens eine Verbindungselement (13, 14) so angeordnet sind, dass ein erster Hohlraum des ersten Hohlelements (10) und ein zweiter Hohlraum des zweiten Hohlelements (11) einen Fließkanal (18) für die Flüssigkeit ausbilden und das mindestens eine Verbindungselement (13, 14) mit dem ersten Hohlelement (10) und/oder mit dem zweiten Hohlelement (11) axial überlappt, wobei ein Formgedächtnis des Formgedächtnismaterials (22) des mindestens einen Verbindungselementes (13, 14) zur Erzeugung einer mechanische Spannung in dem Formgedächtnismaterial aktiviert ist, wobei das mindestens eine Verbindungselement (13, 14) zumindest einen Teil der mechanischen Spannung des Formgedächtnismaterials (22) auf das erste Hohlelement (10) und/oder auf das zweite Hohlelement (11) zum Verbinden des ersten Hohlelements (12) mit dem zweiten Hohlelement (11) überträgt.

15. Implantierbare Anordnung (3) nach Anspruch 14, **dadurch gekennzeichnet, dass** sie unter Anwendung eines Verfahrens nach einem der Ansprüche 1 bis 13 hergestellt worden ist.

16. Blutpumpensystem, umfassend eine implantierbare Anordnung (3) gemäß einem der Ansprüche 14 oder 15 und eine mit der Anordnung (3) verbundene oder verbindbare Blutpumpe zum Fördern von Blut durch den Fließkanal (18) der Anordnung (3).
